# EUROPEAN PATENT APPLICATION

(11) **EP 2 241 633 A1**
(43) Date of publication of application: **20.10.2010**
(21) Application number: 09450079.0
(22) Date of filing: 14.04.2009
(51) Int. Cl.: C12Q 1/48

(54) **Method of determining the activity of PKC**

(71) Applicant: Medizinische Universität Innsbruck, 6020 Innsbruck (AT)
(72) Inventor: Baier, Gottfried, 6020 Innsbruck (AT); Gruber, Thomas, 6364 Brixen i.T. (AT)
(74) Representative: Sonn & Partner Patentanwälte

(57) **Abstract**

The present invention relates to a method of determining the activity of a protein kinase C (PKC) in a cell or cells of a sample comprising determining the amount of Cbl-b in the cell or cells and correlating the Cbl-b amount to the PKC activity.

## Description

The present invention relates to the field of enzymatic activity assays.

The main function of mature T cells is to recognize and respond to foreign antigens. This occurs through a complex activation process that involves activation, adhesion, and differentiation of the resting cell into a proliferating lymphoblast that actively secretes immunoregulatory lymphokines or displays targeted cytotoxicity. Ultimately, this results in the recruitment of other cell types and the initiation of an effective immune response. The activation, fate, and outcome of such induced effector functions of the clonotypic cell are largely controlled by direct cell-to-cell contact to mediate an exchange of information via antigen and adhesion receptors as well as costimulatory molecules. The antigen-specific interaction of a T cell with an antigen-presenting cell (APC) results in the formation of an immunological synapse between the membranes of the two cells. The formation of this functional signaling moiety triggers signaling pathways that involve molecules such as protein kinases, protein phosphatases, and small GTPases. In T cells, this activation cycle includes cytoskeletal remodeling and β2-integrin-mediated cell adhesion. These events transactivate key transcription factors like NFAT (nuclear factor of activated T cells), AP-1 (activator protein-1), and NF-κB (nuclear factor κB) during the course of sustained T cell activation. Subsequently, immediate-early genes like cytokines are expressed, e.g. regulating T cells during clonotypic expansion.

Recruitment of PKC isotypes as amplifying kinases modulates signal strength to overcome negative regulators of cellular signaling. Sustained activation of transcription factors and subsequent cytokine amplification signaling allows entry into S-phase of the cell cycle and promotes cell survival, resulting in many rounds of proliferation of the clonotypic T cells.

Functional analysis of PKC family members in the complex signaling pathways downstream of the T cell receptor (TCR) and CD28 co-receptor has greatly advanced understanding of the unique aspects of T cell type-selective signaling pathways. Employing mostly PKC-isotype selective knockout mice, physiological functions within the transactivation pathways of NF-κB, NFAT, AP-1, and recently, β2-intregrin mediated T cell adhesion were delineated.

PKC isotypes are members of the serine/threonine protein kinase family. PKCs can be classified as conventional PKC isotypes α, β, and γ; the novel PKCs δ, ε, η, and θ; and the atypical PKCs ζ and _{L}. Conventional PKCs require Ca²⁺ and diacylglycerol (DAG) for activation; novel PKCs are Ca²⁺-independent; and atypical PKCs require neither Ca²⁺ nor DAG for activation. Some of the isotypes exist as different splice variants. PKC function is regulated by transphosphorylation and autophosphorylation as well as by the translocation of PKC from one cellular compartment to another, where lipid activators and proteins that bind to the activated form of the enzyme are in proximity to PKC substrates (Baier and Wagner, Current Opinion in Cell Biology 2009, 21: Online Publication DOI 10.1016/j.ceb.2008.12.008).

PKC is an attractive target for modulation of the adaptive immune response; PKC mice were studied in several in vivo disease models for immune-related disorders. These studies showed that PKC mice fail to develop experimental allergic encephalomyelitis, display drastically reduced lung inflammation after induction of allergic asthma, and have a significantly diminished response in experimental colitis and a type II collagen-induced arthritis model. Of note, PKC mice may still mount a normal protective Th1 immune response to clear viral infections. Taken together, this validates PKC as a particularly attractive target for developing ways to selectively manipulate T cell effector functions that are relevant to pathogenesis of different diseases, including asthma, rheumatoid arthritis, and multiple sclerosis.

The most successful of the initial PKC inhibitors is LY333531, a specific inhibitor of PKC β, which is currently being developed for diabetic complications. More recently, the search for PKC inhibitors with various specificities was intensified, guided by the X-ray crystal structures of the catalytic domains of PKC β and θ. Interestingly, selective PKC θ-specific inhibitors have been reported, but no clinical trials have started yet.

A major advance was achieved with AEB071, a very potent and selective low molecular weight inhibitor of both novel and classical PKCs. AEB071 has a greatly improved target selectivity profile in vitro. Current biochemical and pharmacological characterization shows that AEB071 inhibits both of the crucial cytokines, IL-2 and IFN-γ, and surface expression markers (e.g. CD25), effects that are reminiscent of the phenotypes of the PKCα and PKCθ single and double knockout mice. Preclinical studies have shown that AEB071 prolongs rat heterotopic heart transplant survival and cynomolgus monkey renal allograft survival when administered as an oral monotherapy or in combination with adjunct immunosuppressive agents. Furthermore, AEB071 blocks T cell activation through a mechanism independent of calcineurin inhibitors, which are the current standard therapy. Thus, AEB071 may lack the toxicities associated with inhibition of the calcineurin pathway.

PKC inhibitor candidates are usually screened for secondary markers, which are indirectly influenced by PKC activity. Such marker parameters are e.g. interleukin expression or T cell proliferation (Skvara et al., 2008, The Journal of Clinical Investigation 118 (9): 3151-3159). However, measuring T cell proliferation is a relatively laborious assay, which might also be influenced by various other factors. Likewise, the measurement of chemokines and interleukins might suffer from other crossreactivities independent of PKC inhibition. However, up to now, there is no established assay to measure the PKC activity directly in intact cells. It is therefore a goal of the present invention to provide new assays for the determination of PKC activity which can be easily implemented and provide for a signal related to a direct consequence of PKC activity.

Therefore, the present invention provides a method of determining the activity of a protein kinase C (PKC) in a cell or cells of a sample comprising determining the amount of Cbl-b in the cell or cells and correlating the Cbl-b amount to the PKC activity.

The RING-type E3 ubiquitin ligase Cbl-b is a member of the family of casitas B-lineage lymphoma (Cbl) adaptors, which have emerged as potent negative regulators of various signaling cascades in a wide range of cell types. Cbl-b functions as a mediator of antigen-specific T cell intrinsic peripheral immunotolerance, also called clonal anergy. Maintaining the balance between anergy and activation is critical for controlling the expansion of self-reactive T cells involved in immunopathologies. Loss of the Cbl-b gene (cblb) in mice results in exacerbated autoimmunity, anti-viral immunity, and amplified antitumor immunity. Mechanistically, Cbl-b restricts antigen receptor activation by controlling key molecules of T cell activation.

Cbl-b activity is negatively controlled in order to induce and maintain the IL-2-producing capacity of T cells upon cognate TCR and CD28 stimulation. Accordingly, Cbl-b protein levels are posttranslationally downregulated in response to co-receptor CD28 costimulation through ubiquitination and proteasomal degradation. Recently, this down-regulation of Cbl-b was shown to selectively depend on the E3 ubiquitin ligase Nedd4. Nevertheless, the detailed biochemical mechanism for this CD28 engagement-associated Cbl-b regulation remained unknown. In T lymphocytes, PKCθ has been demonstrated to be critical for cytokine responses in vitro and T cell immune responses in vivo, including Th17-mediated autoimmunity. Loss of PKCθ leads to the induction of T cell tolerance, with a phenotype that is comparable to CD28-deficiency. According to the present invention, the essential and non-redundant regulatory function for PKC in the ubiquitination and subsequent degradation of Cbl-b as a prerequisite for an immune response has been identified.

Thus, the present invention has established that the Cbl-b amount or concentration (including a Cbl-b-dependant measurable signal created from the sample) in a sample is a direct function of the PKC activity (which leads to Cbl-b degradation). It is possible to correlate the reduction of the Cbl-b amount to PKC activity. PKC activity is the result of active (not inhibited or inactivated) PKC protein in the cell or cells. Thus the amount of active PKC protein can be correlated to the detected amount of Cbl-b. "Amount" can be understood as the total protein content in the sample or also as the concentration. The inventive correlation is the comparison of a measured Cbl-b amount with a PKC activity. Known PKC activities and respective Cbl-b amount can be used to determine the PKC activity in a sample via the Cbl-b amount. When analysing biological samples, such as cells, it is also possible to use internal or external standards to reference values for the determination of the Cbl-b amounts and its correlation to the PKC activity. Thus, in a preferred embodiment of the present invention the step of correlating the Cbl-b amount to the PKC activity comprises determining standard values of different PKC activities and the resulting Cbl-b amount in a cell or cells and comparing the standard values with the Cbl-b amount of the sample. For the sake of providing PKC activity references it is e.g. possible to stimulate PKC, such as by CD28. It is also possible to perform the inventive test method on a sample and in addition on a sample with a known PKC inhibitor, which for example completely or to a known degree reduces PKC activity.

Of course, as the skilled man in the art will readily appreciate, it is conceivable to use standards to determine the Cbl-b amount itself by any known methods. E.g., it is possible to use cells, which contain a known Cbl-b amount that can then be used to correlate a given Cbl-b dependant signal of the standard to a signal in the cells of the sample. The determined Cbl-b amount in turn is used to determine the PKC activity.

The cells used in the inventive methods may be any type of cells but preferably comprise eukaryotic cells, such as of a bird or mammal, including a rodent or primate, in particular cells of a human, mouse, rat, hamster, chimpanzee or pig. For some uses of the inventive method it might be suitable to select specific classes of cells from any organism, such as cells of the immune system, if the effect of the PKC activity is to be analysed in such cells. For example, these cells might be used to screen for immunomodulating substances, which modify the PKC activity in these cells. Therefore, preferred cells include PBMCs (peripheral blood mononuclear cells), in particular leukocytes (T-lymphocytes, B-lymphocytes, NK-cells, NKT-cells, monocytes, macrophages and/or dendritic cells), in particular CD8+ T-lymphocytes, CD4+ T-lymphocytes, such as Th1, Th2, Th17, Tregs (regulatory T cells). Some cells might be selected for further subpopulations characterized by a given surface marker such as CD4, CD8, CD25, CD69, CD70, CD27, CD39, CD54, CD45RA, CD45RO, CD62L, CD73, CD95, CD107a, CD127, CD134, CDw137, CD152, CD154, CCR4, CCR6, CCR7, CCR8, CXCR3, GITR, PD-1, A2AR, a cytokines such as IL-2, IL-6, IL-7, IL-10, IL-15, IL-17A, IL-17F, IL-21, IL-22, IL-26, IL-27, Interferon-γ, Lymphotoxin-α, TNF-α, and intracellular molecules, in particular Foxp3, GATA-3, RORc, T-bet.

In some embodiments of the present invention determining the Cbl-b amount comprises determining the amount of Cbl-b mRNA. It is either possible to determine the amount of Cbl-b mRNA as internal standard to e.g. determine differences to the Cbl-b (protein) amount or directly as analyte in order to assess any effects thereon, in particular to estimate a down- or upregulation. Differences, or the ratio between Cbl-b mRNA and Cbl-b protein amounts (e.g. as compared to the normal mRNA/protein ration) can indicate strong degradation of Cbl-b protein. In pratice, the mRNA determination can be performed by any known methods, including PCR based assays or hybridisation based detection methods. In another preferred embodiment determining the Cbl-b amount comprises determining the amount of Cbl-b protein. It is also possible to specifically determine ubiquitinated Cbl-b protein or phosphorylated Cbl-b protein. According to the present invention it was found that Cbl-b protein amount or the fraction of ubiquitinated or phosphorylated Cbl-b is a direct consequence of PKC activity. It was shown that inhibition of the enzymatic activity of PKC-theta isotype decreases the level of ubiquitinated Cbl-b and thus leads to an increased Cbl-b protein concentration in cells. Ubiquitinated Cbl-b will be readily removed by ubiquitin-dependent proteasomal degradation. According to the present invention it is possible to simply determine the total Cbl-b amount and it is also possible to specifically determine subfractions of Cbl-b, such as ubiquitinated Cbl-b and/or phosphorylated Cbl-b. Furthermore, it was surprisingly found that Cbl-b ubiquitination occurs as a direct result of PKC activity. It was also found, that Cbl-b is phosphorylated by PKC-theta, and thus creating a subfraction of Cbl-b which concentration and fraction directly correlates to PKC activity. In particular, PKC-theta readily phosphorylated Cbl-b at SER-282 but other phosphorylation sites also exist. For example, ubiquitinated or phosphorylated Cbl-b can be distinguished by use of ubiquitin or phosphor labels (in particular radio or fluorescence labels), which can be attached to Cbl-b via the cells own protein processing systems, or using antibodies specific for ubiquitinated or phosphorylated Cbl-b.

In preferred embodiments the cells or cell of the sample are contacted with a drug candidate before or during the Cbl-b amount is determined. A particular class of drugs that may be screened for with the inventive methods are PKC inhibitors, which have a potential anti-inflammatory action. Inhibition of PKC will lead to a reduced degradation of Cbl-b and thus, increased Cbl-b amount. On the other hand, it is also possible to screen for drugs that augment or enhance PKC activity, which will in turn lead to a decreased Cbl-b amount. As used herein, drugs are active pharmaceutical agents, which have an effect on PKC and modulate its activity. Such agents include organic molecules, in particular small organic molecules, biomolecules such as protein or nucleic acids (inhibitory nucleic acids, e.g. antisense or SiRNA, or agonizing nucleic acids such as nucleic acids that encode for proteins with PKC activity). Enhancers and agonists are for example also proteins with PKC activities, such as PKC itself. Preferred isoforms of PKC that are analysed with the inventive method are conventional PKC, such as PKC-β, or a novel PKC, such as PKC-theta. There exist some PKC inhibitors that might affect all or many PKC isoforms (pan-PKC inhibitors) but also those that modulate a single isoform. One of the preferred PKC isoforms is PKC-theta.

The Cbl-b amount measured after contact with the drug candidate can be correlated to an increase or decrease of PKC activity due to the drug candidate in reference to a cell or cells without contact to the drug candidate. Thus, it is possible to directly determine and quantity the drugs potency on the increase or decrease of the PKC activity.

In particular preferred embodiments intracellular Cbl-b amount is determined. There exist several methods in the art to directly or indirectly determine intracellular analytes. Such intracellular determination methods usually include a step of fixation to prevent the fusion of the analyte out of the cells. Such fixation methods are particularly advised, if the cells are permeabilised in order to introduce a labelled moiety into the cell that will give the Cbl-b-dependent signal. Such moieties are, e.g., labelled antibodies. During the step of cell fixation usually the cell content is crosslinked with a crosslinking reagent, which forms a stable network scaffold of proteins and nucleic acids contained in the cell. Such a crosslinking reagent is, for example, formaldehyde. The antibody used for the intracellular determination of Cbl-b is usually suitable to bind to the crosslinked or fixed form of Cbl-b. Such an antibody is, e.g., antibody Abcam Ab54362 (commercially available from Abcam), which recognizes a C-terminal portion of Cbl-b. In general, a preferred antibody that is used for the intracellular determination of Cbl-b is, e.g., an antibody that recognizes an epitope within the C-terminal 300 amino acids of Cbl-b, preferably within the C-terminal 250 amino acids of Cbl-b. In particular preferred is an antibody that recognizes amino acids 833 to the C-terminus of Cbl-b or an epitope within amino acids 833 to 964 of Cbl-b. Such an antibody might be of any origin as long as it can bind the Cbl-b target or epitope of Cbl-b. The antibody might be a human, humanized, mouse, rat, hamster, pig or primate antibody. It can be monoclonal or polyclonal.

In further preferred embodiments of the invention the amount of Cbl-b is determined in single cells, preferably by flow-cytometry. Using such methods it is possible to correlate a Cbl-b-dependent signal to a single cell and it is thus possible to differentiate between different types of cells. In particular, it is conceivable that different cells of the sample react differently to a given PKC modulating drug candidate. For example, it is possible that Th17 cells might give a relevant signal but not Th1 cells. In this example, by using a method that can determine Cbl-b in single cells, it is possible to identify a significant immunological response related Th17 cells caused by the PKC inhibitor. In methods where only the average of all cells in the sample is measured it could happen that this average value would not lead to the identification of a potent drug candidate. Of course it is also possible to specifically isolate given cell types as mentioned above by common cell purification methods and then determine the Cbl-b amount in this sub population of cells. Still, a method that can quickly determine Cbl-b amounts in Cbl-cells such as flow-cytometry are preferred since no pre-purification of the cells is necessary and each cell dependent signal can be associated to a given cell population, e.g. using a multi-coloric method wherein a second signal (second colour signal) is created dependent on other cell surface markers distinctive for said cell type or class (e.g. using a labelled anti-CD4 antibody to screen for CD4⁺ T cells with a second color signal).

In another aspect, the present invention provides a method to evaluate, monitor or prognose the efficacy of a PKC-theta-modulator therapy in a patient. Due to the cross-talk between PKC-theta and Cbl-b it is possible to determine the prospect and progression of such a therapy. The PKC-theta modulator can be an inhibitor (or antagonist) or an agonist. This method usually comprises determining the amount of Cbl-b in a cell or cells in a sample of the patient to whom the PKC-theta-inhibitor is administered. The calls are preferably cells of the immune systems as mentioned previously. Preferably, the Cbl-b is determined according to any method disclosed above. "Monitoring" should be understood as a repeated determination of the Cbl-b amount in order to determine any changes in the Cbl-b amount and thus consequently in the PKC-theta activity. By determining the Cbl-b amount it is possible to prognose and evaluate the PKC-theta activity in the cell or cells and thus, determine the outlook or progression of the therapy in response to a PKC-theta inhibitor. The patient may be any patient to whom a PKC-theta-modulator might be administered or who is currently undergoing a PKC-theta-modulator therapy. This therapy comprises the administration of a PKC-theta-modulator, e.g. inhibitors as mentioned above or identified by the inventive method or agonists such as PKC-theta itself. The patient might suffer from any diseases associated with an abnormal PKC-theta activity mentioned herein, in particular inflammatory and/or immunological diseases, such as Th17-mediated inflammatory or immunological diseases, including autoimmune disorders, or a Th1 and/or Th2-mediated allergic inflammation, in particular autoimmune encephalitis, multiple sclerosis, arthritis, transplant rejection, inflammatory bowel disease or psoriasis.

In preferred embodiments of the present invention the inventive method of determining PKC activity by way of Cbl-b amount determination is used to identify drugs for the treatment of inflammatory and/or immunological diseases, preferably Th17-mediated inflammatory or diseases such as autoimmune disorders or a Th1 and/or Th2-mediated allergic inflammation, in particular autoimmune encephalitis, multiple sclerosis, arthritis, transplant rejection, inflammatory bowel disease, psoriasis.

As stated in the introduction, PKC, in particular PKC-theta is a central regulator of regulatory immune responses and is a current drug target to treat diverse inflammatory and autoimmune conditions. Therefore the invention relates also to a therapy of an inflammatory (including autoimmune-) disease using the identified PKC inhibiting drug.

The present invention is further illustrated by the following figures and examples, without being restricted thereto.

### F I G U R E S:

### Figure Legends

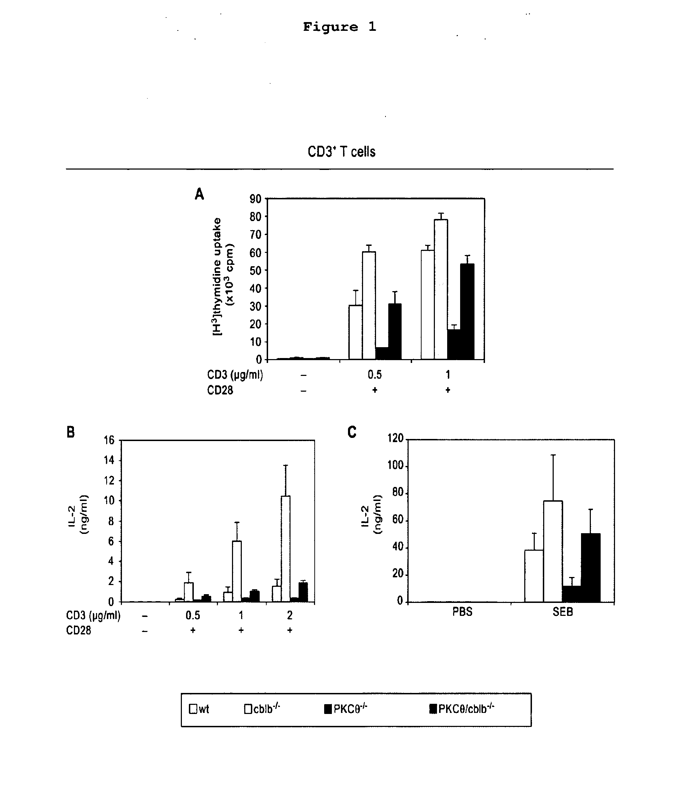

**Fig. 1****: Impaired activation of PKC**θ**-deficient T cells is rescued by deletion of Cbl-b.**
   (A, B) CD3⁺ cells were stimulated with low concentrations of anti-CD3/anti-CD28, as indicated. Of note, differences between genotypes decreased upon using concentrations as high as 10 µg/ml anti-CD3. (C) CD4⁺ cells were stimulated with the superantigen Staphylococcus enterotoxin B (SEB) together with syngeneic antigen presenting cells (APCs). Proliferation was measured via incorporation of ³H-thymidine (A), and IL-2 in the supernatants was quantified by Bioplex technology (B, C). (ANOVA, PKCθ^{-/-} vs. PKCθ^{-/-}/cblb^{-/-}, p=0.0002 (A); p<0.0001 (B);
   p=0.01 (C)) Results shown are the means ± SD of three independent experiments.

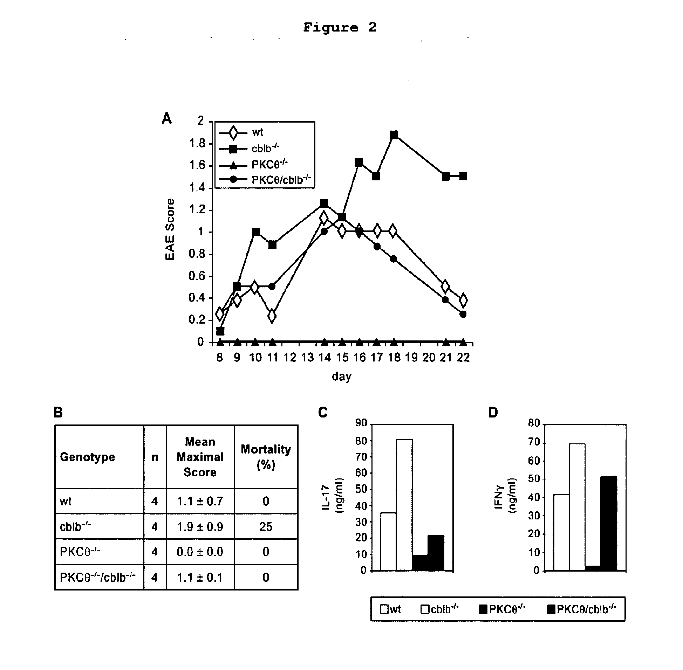

**Fig. 2****: Loss of Cbl-b restores EAE susceptibility of PKC**θ-**deficient mice.**
   (A, B) On day 1, mice were injected with 200 µg MOG35-55 followed by two injections of 200 ng pertussis toxin on days 2 and 3. Thereafter, mice were checked for symptoms of paralysis, expressed as severity scores (ranging from 0, no abnormalities, to 4, animal dead or moribund). One of two experiments with similar outcomes is shown. (C+D) IL-17 and IFNγ responses of ex vivo differentiated Th17 cells. One of two experiments with similar outcomes is shown.

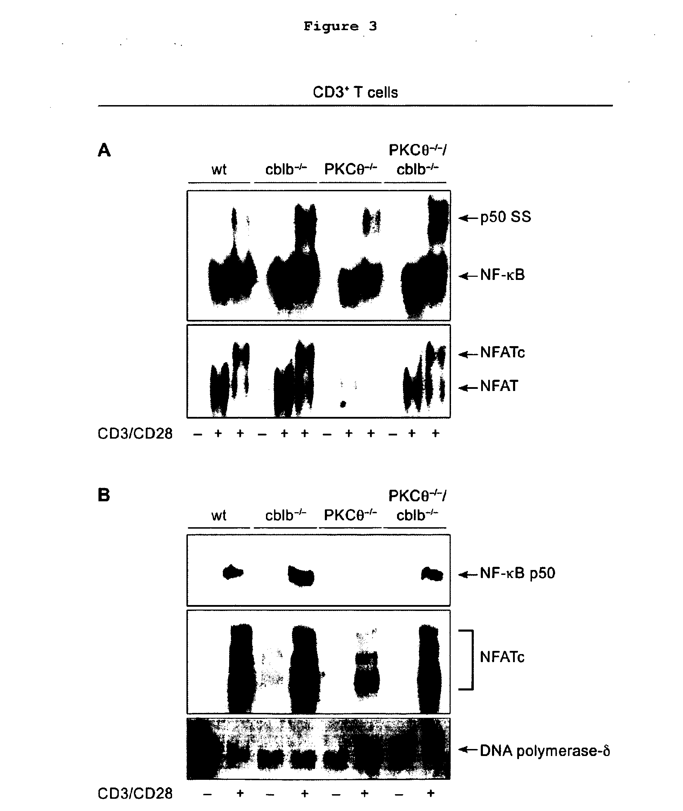

**Fig. 3****: The PKC**θ**-dependent activation of the critical transcription factors NF-κB and NFAT is restored to wild type levels in PKCθ/Cbl-b double knockout T cells.**
   CD3⁺ T cells were stimulated with anti-CD3 and anti-CD28 for 18 h. Nuclear extracts were analyzed by electrophoretic mobility shift assay (EMSA) to assess DNA binding (A) or on immunoblots to measure nuclear entry (B). Cytosolic levels of NFATc and p50 NF-κB in CD3⁺ T cells remained unchanged between the different genotypes. Equal protein amounts were used for EMSA analysis and immunoblots. DNA polymerase δ in the nuclear extracts served as a loading control.

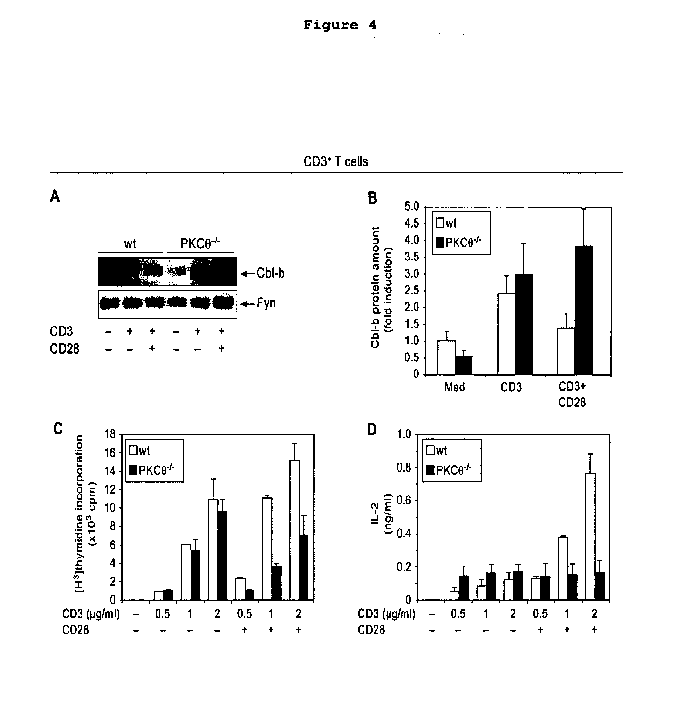

**Fig. 4****: Costimulation-dependent degradation of Cbl-b is impaired in PKC**θ**-deficient T cells.**
   (A, B) CD3⁺ T cells were stimulated overnight, rested, and restimulated as indicated. Cell extracts were immunoblotted for Cbl-b and Fyn (loading control; A) and relative amounts of Cbl-b protein was quantified by densitometry (B; ANOVA, n=5, wt vs. PKCθ^{-/-}, p=0.035). (C, D) Proliferation and IL-2 activation response of PKCθ^{-/-} T cells upon CD3 single and CD3/CD28 double stimulation conditions. Results shown are the means ± SD of two independent experiments.

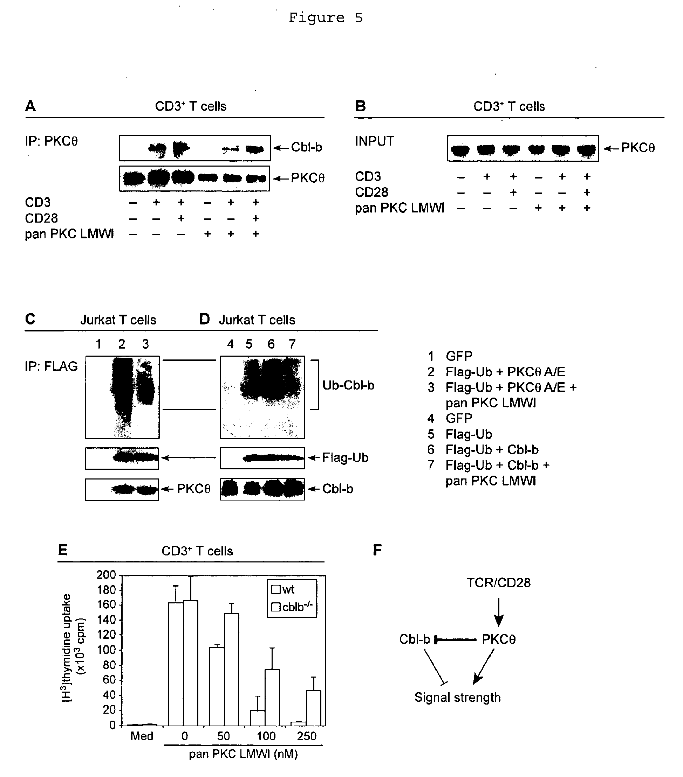

**Fig. 5****: Cbl-b associates with PKCθ upon short-term TCR stimulation. (A)** CD3⁺ T cells were stimulated for 15 min employing anti-CD3, anti-CD28 and crosslinking anti-hamster secondary antibodies, as indicated. Lysates were immunoprecipitated with anti-PKCθ and blotted for Cbl-b and PKCθ. Cells were pretreated with DMSO carrier or panPKC inhibitor, as indicated. One out of two experiments with similar outcomes is shown. Of note, the PKCθ/Cbl-b complex was not immunoprecipitated by the stimulatory antibodies added to post lysis extracts, excluding the artefact of detecting CD3/CD28-associated proteins via these stimulating antibodies (not shown). (**B**) Input control of PKCθ in whole cell extracts used for coimmunoprecipitation analysis. (C, D) Ubiquitination of Cbl-b is dependent on catalytically active PKCθ. Jurkat T cells were transfected with the indicated constructs encoding human Cbl-b and the constitutive active mutant A148E of PKCθ (PKCθA/E) and stimulated with 10 µg/ml anti-CD3 for 90 min after preincubation with 50 µM proteasome inhibitor MG132 for 1 h. Where indicated, 1 µM panPKC inhibitor was added. Lysates were immunoprecipitated with anti-Flag and blotted with anti-Cbl-b. (E) Proliferative responses of panPKC inhibitor-treated CD3+ T cells are partially restored by the loss of Cbl-b. CD3+ T cells were stimulated with 10 µg/ml anti-CD3 plus 1 µg/ml CD28 and treated with the indicated concentrations of panPKC inhibitor. Results shown are the means ± SD of two independent experiments. (F) Proposed model: Cbl-b negatively controls signal strength of antigen-specific immune responses. TCR/CD28 signaling induces PKCθ-mediated inactivation of Cbl-b as prerequisite of a productive T cell immune response.

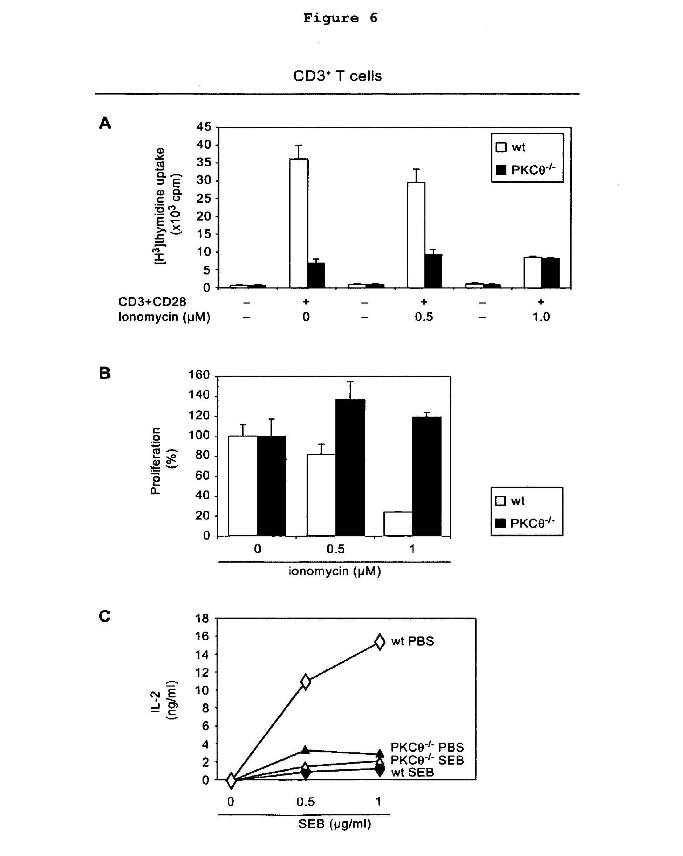

**Fig. 6****: PKCθ^{-/-} T cells exhibit hyporesponsiveness.**
   (A) Anergy induced by Ca²⁺ ionophore pre-treatment inhibits the proliferation response in mouse CD3⁺ T cells induced by CD3/CD28 stimulation. Following washout, wild type CD3⁺ T cells in solvent alone responded strongly to CD3/CD28 stimulation but they were increasingly unresponsive as the ionomycin concentration was increased. In contrast, PKCθ^{-/-} CD3⁺ cells already responded poorly to CD3/CD28 stimulation and this hyporesponsiveness was not affected further by increasing concentrations of ionomycin. Of note, defective proliferation of PKCθ^{-/-} T cells can be mostly rescued by adding exogenous IL-2. (B) The response of non-treated cells was set = 100%. PKCθ^{-/-} CD3⁺ cells already proliferated poorly prior to anergy induction; proliferation was not reduced further by ionomycin treatment. All assays were performed in duplicate. Results shown are the means ±SD of three independent experiments. (C) Anergy induced by SEB in vivo inhibits the IL-2 response of ex vivo restimulated CD4⁺ T cells. Cells from SEB pretreated wild type mice were energized efficiently, whereas cells from PKCθ^{-/-} mice already responded poorly after PBS treatment, and this hyporesponsiveness was not further affected by SEB pretreatment.

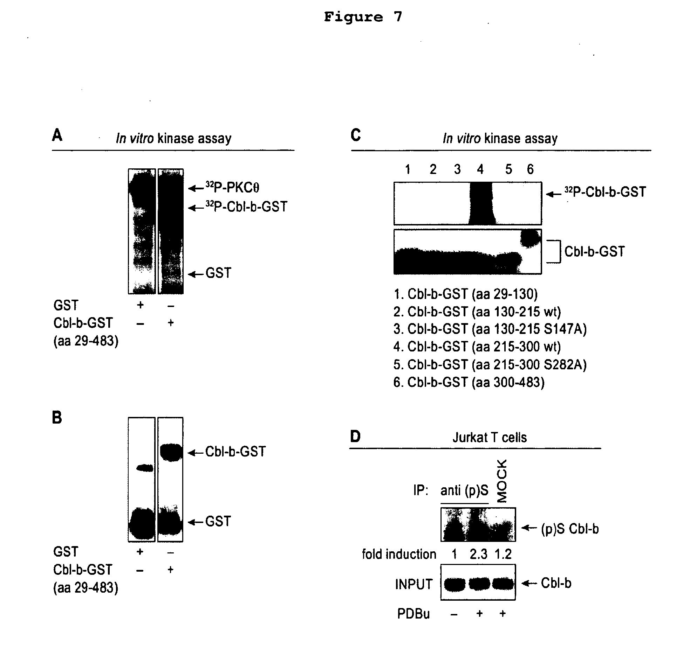

**Fig. 7****: PKC**θ **is able to phosphorylate the TKB domain of Cbl-b in vitro.**
   (A-C) Kinase assay of GST-Cbl-b subdomains incubated with recombinant PKCθ. Specific phosphorylation of GST-Cbl-b TKB domain (NH₂-terminal region of Cbl-b ranging form aa 29-483) and particularly of Ser 282 on the GST-Cbl-b subdomain, ranging form aa 215-300 (lane 4) was reproducibly detected. In contrast, no phosphorylation of the S282A mutant fusion protein (lane 5) nor S147 (another bioinformatically identified PKC site within subdomain ranging from aa 130-215) or GST alone could be observed. Of note, however, upon long exposure additional phosphorylation by PKCθ could be observed in both the Cbl-b subdomains ranging from aa 29-130 and aa 300-483. These additional sites, however, remain unmapped. The anti-GST immunoblots confirmed equal loading (B & lower panel in C). Experiments were repeated at least two times, with similar results.
   (D) Serine-phosphostatus analysis of Cbl-b in intact T cells. Serine-phosphorylated Cbl-b was immunoprecipitated with a broadly reactive phospho-Ser antibody. Stimulation with PDBu (a pleiotropic PKC agonist) of Jurkat T cells led to a 2.3 fold higher reactivity of this (p)Ser antibody with Cbl-b (upper panel). The input control for Cbl-b (bottom panel) is shown.

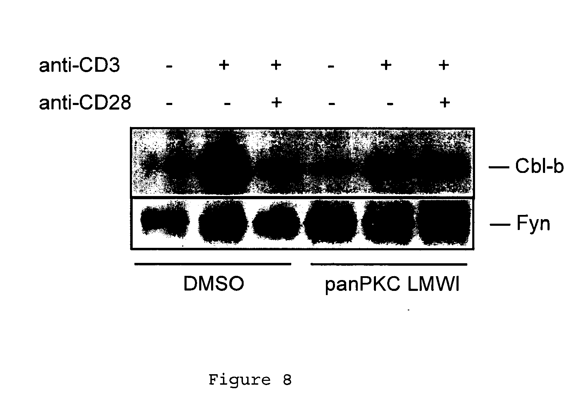

**Fig. 8****: Inhibition of PKCθ abolishes CD3-CD28 induced Cbl-b degradation**
   Importantly, enzymatically inhibited PKCθ almost completely abolished CD3-CD28 induced Cbl-b degradation in primary CD3+ T cells, clearly indicating that the enzymatic activity and not a simple scaffold function of PKC is essential for inducing Cbl-b ubiquitination in T cells. DMSO solvent control or panPKC inhibitor-treated cells were stimulated by CD3 or CD3/CD28 agonistic antibodies, as indicated. This result is consistent with the impaired Cbl-b degradation in *PKC*θ^{-/}*⁻ T* cells (Fig. 4A&B , independently confirming that PKCθ functions as upstream repressor of Cbl-b by regulating Cbl-b ubiquitination and degradation.

### EXAMPLES:

### Example 1: Material and Methods

### Example 1.1: cblb knockout mice and reagents

PKCθ and cblb knockout mice were described elsewhere. The panPKC low molecular weight inhibitor (LMWI) was provided by Altana-Pharma (Konstanz) and is described.
γ³²P-ATP was purchased from Amersham. Phorbol 12,13-Dibutyrate (PDBu) and ionomycin were from Sigma (Vienna, Austria). The antibodies used for T cell stimulation were anti-CD28 mAb (clone 28.2) and the CD3-specific OKT3 (human) mAb and 2C11 (mouse) mAb. The broadly reactive phospho-Ser (clone 7F12) mAb was from Biomol. The human PKCθ, Cbl-b and 3xFlag-tagged ubiquitin expression plasmids where cloned under the control of EF1α-promoter within pEF-neo. The constitutive active mutant PKCθ A148E (A/E) has been described previously.

### Example 1.2: Analysis of proliferation responses and cytokine release

Mature CD3⁺ T cells were purified from pooled spleen and lymph nodes using mouse T cell enrichment columns via negative selection (R&D Systems). T cell populations were typically 95% CD3⁺ as determined by staining and flow cytometry. For anti-CD3 stimulation, 5 × 10⁵ T cells in 200 µl proliferation medium (RPMI supplemented with 10% fetal calf serum [FCS], 2 mM L-glutamine, and 50 units ml⁻¹ penicillin/streptomycin) were added in duplicate to 96-well plates precoated with indicated concentrations of anti-CD3 antibody. Soluble 1 µg ml⁻¹ anti-CD28 was also added. For tolerance induction, cells were pretreated with ionomycin for 16 h. Cells were harvested at 40 h after a 16 h pulse with 1 µCi [³H]-thymidine per well; [³H]-thymidine incorporation was measured with a Matrix 96 direct β counter system. Alternatively, mature CD4⁺ T cells and antigen producing cells (APCs) were selected from pooled spleen and lymph nodes with magnetic beads (Miltenyi Biotec). Subsequently, 5 × 10⁵ T cells were stimulated with 5 × 10⁵ SEB-pulsed APCs (10 µg/ml SEB) and harvested at 64 h. IL-2 from culture supernatants of CD3⁺ or CD4⁺ T cells was measured by BioPlex technology (BioRad). In vivo anergy induction with SEB was performed as described.

### Example 1.3: Induction of experimental autoimmune encephalomyelitis

MOG₃₅₋₅₅ peptide was synthesized by NeoSystems, France. Eight to 12-week old mice were immunized with one injection of 200 µg MOG₃₅₋₅₅ peptide (in CFA supplemented with 5 µg ml⁻¹ *Mycobacterium tuberculosis* H37 Ra [Difco Laboratories], 1:1 in phosphate buffered saline [PBS]) in a total of 200 µl into the hind flank. In addition, 200 ng of pertussis toxin (Sigma Aldrich) dissolved in 200 µl PBS was injected into the tail veins 24 and 72 h later. Mice were monitored daily for clinical signs of EAE and graded from 0 to 4 on a scale of increasing severity by two independent investigators.

### Example 1.4: Antigen recall assay

On day 22, splenocyte suspensions were isolated from MOG₃₅₋₅₅ peptide immunized mice along with PBS treated control mice. Splenocytes from individual mice, depleted of RBC with lysing buffer (R&D), were plated in duplicates (5x10⁵/well) in 200 µl proliferation medium (RPMI supplemented with 10% FCS, 2 mM L-glutamine and 50 U ml⁻¹ penicillin/streptomycin) containing either 0, 10 or 100 µg ml⁻¹ MOG₃₅₋₅₅ peptide and cultured at 37°C in 5% CO₂. Cells were harvested at 90 h after a 16 h pulse with 1 µCi [³H]-thymidine per well; [³H]-thymidine incorporation was measured with a Matrix 96 direct β counter system.

### Example 1.5: Th17 in vitro differentiation

For T cell differentiation naive CD4⁺ cells were isolated via the CD4⁺ CD62L⁺ T Cell Isolation Kit II (Miltenyi Biotec). Polarization of T cells into Th17 cells was performed by solid-phase anti-CD3 (5µg/ml) and soluble anti-CD28 (1µg/ml) in the presence of polarizing cytokines (10ng/ml IL-23, 5 ng/ml TGF-β, 20 ng/ml IL-6 and 2 µg/ml of both anti-IL-4 & anti-IFN-γ as described (Yang et al., Immunity 28 (2008): 29-39). Supernatant was collected on day 4 and analyzed via BioPlex multi-analyte technology (BioRad).

### Example 1.6: Gel mobility shift assays

Nuclear extracts were harvested from 2 × 10⁷ cells according to standard protocols. Briefly, CD3⁺ T cells were purified, washed in PBS, and resuspended in 10 mM HEPES, pH 7.9, 10 mM KC1, 0.1 mM EDTA, 0.1 mM EGTA, 1 mM DTT, and protease inhibitors. Cells were incubated on ice for 15 min. NP-40 was added to a final concentration of 0.6%, the cells were vigorously mixed, and the mixture was centrifuged for 5 min. The nuclear pellets were washed twice and resuspended in 20 mM HEPES, pH 7.9, 0.4 M NaCl, 1 mM EDTA, 1 mM EGTA, 1 mM DTT, 12 and protease inhibitors, and the tubes were rocked for 30 min at 4°C. After centrifugation for 10 min, the supernatants were collected. 2 µg extract proteins were incubated in binding buffer with end-labelled, double-stranded oligonucleotide probes (NF-κB: 5'-CTG GGG ACT TTC CGC T-3'; NFAT: 5'-GCC CAA AGA GGA AAA TTT GTT TCA TAC AG-3'). 3 × 10⁵ cpm of labelled probe was used in each reaction and band shifts were resolved on 4% and 5% polyacrylamide gels. Where indicated, 0.5 µg of supershift antibody was added. All experiments were performed at least three times with similar outcomes.

### Example 1.7: Protein kinase assay

Recombinant E. coli-expressed GST-Cbl-b fusion proteins were purified using glutathionsepharose (Amersham). PKC-dependent phosphorylation was determined by measuring the amount of ³²Pᵢ incorporated into Cbl-b in a standard kinase assay. Two hundred nanograms of purified recombinant GST-Cbl-b and PKCθ were incubated in kinase assay buffer (40 mM Tris pH 7.5, 40 mM MgCl₂, 0.2 mM DTT, 0.0002% Triton X-100, 0.3 µg ml⁻¹ bovine serum albumin) containing 1 µM ATP, 2 µCi [γ³²P-ATP], 1 µM PDBu, and 160 µM phosphatidylserine. After 20min at 30°C, stop solution was added (10 mM ATP, 5 mM EGTA pH 7.5, 0.1% Triton X-100). Incorporated radioactivity was measured by SDS-PAGE and autoradiography.

### Example 1.8: Flow cytometry

Single cell suspensions were prepared and incubated for 30 min on ice in staining buffer (PBS containing 2% FCS) with fluorescein isothiocyanate, phycoerythrin, and allophycocyanin antibodies. Splenocyte surface marker expression was analyzed using a FACS Calibur™ cytometer (BD Biosciences) and CellQuest™ software according to standard protocols. Antibodies against murine CD3, CD4, and CD8 were obtained from Caltag Laboratories; CD69, CD44, CD25, and Vβ8 were from BD Pharmingen.

### Example 1.9: Western blot analysis

For Cbl-b degradation experiments, CD3⁺ T cells were stimulated with 10 µg/ml solid phase hamster anti-CD3 plus 1 µg/ml hamster anti-CD28 (clone 37.51; BD Biosciences) and 10 U/ml IL-2 at 37°C for 20 h. Subsequently, T cells were rested in 10 U/ml IL-2 overnight and restimulated with 10 µg/ml anti-CD3 with or without 1 µg/ml anti-CD28 for 20 h. Cells were lysed in ice cold lysis buffer (5 mM NaP₂P, 5 mM NaF, 1 mM Na₃VO₄, 5 mM EDTA, 50 mM NaCl, 50 mM Tris, pH 7.3, 2% NP-40, 50 µg/ml each aprotinin and leupeptin) and centrifuged at 15,000 g for 15 min at 4°C. Protein lysates were subjected to western analysis using antibodies against Cbl-b or Fyn (Santa Cruz Biotechnology, Inc.). To detect the transcription factors, nuclear extracts were prepared according to standard protocols and mAb 7A6 for NFATc (Affinity BioReagents, Inc.) and pAb for p50 (Active Motif) were used. As a loading control, pAb against DNA polymerase δ (Santa Cruz Biotechnology, Inc.) was applied.

### Example 1.10: Transfections

Jurkat-TAg cells were maintained in RPMI medium supplemented with 10% FCS (Life Technologies, Inc.) and antibiotics. Transient transfections of 15 µg of PKCθ or Cbl-b plasmids were performed by electroporation (plus either 15 µg of the green fluorescent protein or 15 µg of Flag-tagged ubiquitin expression vectors) using a BTX-T820 Electro Square Porator (ITC, Biotech, Heidelberg, Germany) apparatus under predetermined optimal conditions: 2 × 10⁷ cells at 450 V cm⁻¹ and 5 pulses of 99 ms.

### Example 1.11: Co-immunoprecipitation analysis

A total of 1 × 10⁷ Jurkat T cells were lysed in 400 µl lysis buffer. For ubiquitination assays, 10mM N-ethylmaleimide was added. Lysates were precleared for 1 h at 4°C and incubated with 2
µg of appropriate antibodies (PKCθ, Santa Cruz; Flag, Sigma) at 4°C overnight. Thereafter, incubation with protein G Sepharose (Amersham-Pharmacia, Vienna) for 1 h at 4°C, 5× washing in lysis buffer, and SDS-PAGE under reducing conditions on bis/tris-buffered gels (Novex, San Diego, CA) were performed. Proteins were transferred onto a polyvinyl-difluoridon membrane (Millipore, Bedford, MA) by semi-dry blotting (90 mA, 80 min). The primary Abs were diluted in tris-buffered saline containing 0.5% Tween-20 and 5% non-fat dry milk. Peroxidase conjugated antibodies (Pierce, Rockford, IL) served as secondary reagents (1:5000). Enhanced chemiluminescence was used for antigen detection (Super Signal, Pierce, Rockford, IL).

### Example 1.12: Statistical analysis

Statistical analysis was performed using the statistical package R (www.cran.r-project.org).

### Example 2: Results

### Example 2.1: PKCθ^{-/-} / cblb^{-/-} knockout mice

PKCθ is essential to augment T cell activation as has been shown in several studies. Indeed, and in agreement with Berg-Brown et al., in both in vitro and in vivo models of tolerance induction using Ca²⁺ ionophore or superantigen Staphylococcus enterotoxin B (SEB), respectively, it was found that loss of PKCθ per se induced a hyporesponsive state (Fig. 5A-C). However, the molecular mechanism behind is not known. One study showed that Ca²⁺ flux induced by ionomycin leads to the expression of multiple genes. Considering the contrasting immunophenotypes of PKCθ and Cbl-b, an "anergy factor", knockout mice, it was investigated whether the loss of Cbl-b could rescue any of the defects that have been ascribed to PKCθ-deficiency. Therefore, mice that were deficient in PKCθ and Cbl-b were generated and their immune phenotype was characterized. PKCθ^{-/-}/cblb^{-/-} mice were viable and fertile. Similar to splenocytes from single knockout mice, FACS analysis of PKCθ^{-/-}/cblb^{-/-} splenocytes showed no significant differences in the distribution of CD3, CD4, and CD8-positive cells as compared to those from wild type littermates (Table 1).

**Table 1: Effect of cblb/PKCθ single and double deficiency on cellularity (percentages of surface marker positive cells). Similarly, CD3 and Vβ8 surface marker densities were not altered between genotypes.**

| **Spleen subsets (Mean ± SEM; n=3)** | | | | |
|---|---|---|---|---|
| | wt | *cblb*^{-/-} | *PKC*θ^{-/-} | *PKC*θ/*cblb*^{-/-} |
| CD3⁺ | 41.7 ± 8.2 | 32.2 ± 7.9 | 39.1 ± 3.3 | 30.7 ± 3.0 |
| CD4⁺ | 21.3 ± 0.7 | 17.7 ± 0.1 | 17.2 ± 0.1 | 13.6 ± 1.6 |
| CD8⁺ | 11.6 ± 0.7 | 10.2 ± 0.3 | 12.0 ± 0.7 | 8.6 ± 0.3 |
| Vβ8⁺ | 19.4 ± 2.2 | 20.7 ± 4.4 | 20.8 ± 2.8 | 14.8 ± 3.0 |

When analyzing the activation thresholds of PKCθ and Cbl-b single and double deficient T cells, concomitant loss of Cbl-b substantially restored the proliferation and IL-2 secretion responses induced by either anti-CD3/CD28 or SEB back to wild type levels (Fig. 1A-C). The degree of rescue in double-deficient T cells depended on the strength of CD3/CD28 stimulation employed. Moreover, while PKCθ^{-/-} T cells showed a profound defect in all activation markers analyzed (i.e. CD25, CD44, and CD69), the concomitant loss of Cbl-b restored expression to wild type levels (Table 2).

**Table 2: Surface expression of CD25, CD44, and CD69 activation marker signals on mouse CD3⁺ T cells, expressed as the median fluorescence intensities (MFI±SEM; n=3). Flow-cytometric analysis of T cells, stimulated for 16 h by CD3/CD28 ligation was performed, using fluorescence-labelled antibodies.**

| | **unstimulated** | | | | **stimulated** | | | |
|---|---|---|---|---|---|---|---|---|
| | wt | *cblb*^{-/-} | *PKC*θ^{-/-} | *PKC*θ^{-/-}/*cblb*^{-/-} | wt | *cblb*^{-/-} | *PKC*θ^{-/-} | *PKC*θ^{-/-}/*cblb*^{-/-} |
| CD25 | 3.6 ± 0.2 | 4.0 ± 0.3 | 3.7 ± 0.2 | 3.7 ± 0.4 | 242.2 ± 64.6 | 326.7 ± 106.5 | 143.4 ± 64.7 | 418.1 ± 175.8 |
| CD44 | 59.6 ± 14.4 | 42.2 ± 19.9 | 46.3 ± 15.8 | 50.9 ± 10.7 | 150.6 ± 35.0 | 149.9 ± 48.7 | 90.1 ± 17.6 | 177.7 ± 24.2 |
| CD69 | 4.0 ± 0.4 | 3.8 ± 0.3 | 4.1 ± 0.4 | 3.6 ± 0.4 | 208.9 ± 65.9 | 260.7 ± 97.3 | 143.8 ± 43.6 | 225.3 ± 45.1 |

These rescued activation responses were not due to upregulation of either CD3 or TCR-Vβ8 receptor expression on the surface of T cells (Table 1). To gain more insight into PKCθ/Cbl-b crosstalk in vivo, mice were immunized with the myelin component MOG₃₅₋₅₅ to induce experimental autoimmune encephalomyelitis (EAE), a multiple sclerosis-like autoimmune disease. Consistent with previous studies, PKCθ-deficient mice were insensitive and Cbl-b deficient mice were hypersensitive to the induction of EAE (Fig. 2A&B ). Strikingly however, PKCθ/Cbl-b double knockout mice demonstrated similar onset and overall disease scores as wild type mice when determining progressive paralysis from tail to head (Fig. 2A&B ).

Consistent with disease progression, cblb-deficient T cells exhibited an augmented ex vivo recall response to MOG₃₅₋₅₅, whereas PKCθ^{-/-} T cells showed a profound proliferation defect. Again, the response of the double-deficient T cells was completely restored to wild type levels. It has been shown that the induction of EAE is correlated with the expression of two inflammatory cytokines, i.e. IL-17 and IFNγ. In light of these findings, naive CD4⁺ T cells were isolated from all four genotypes, differentiated them ex vivo into Th17 effector/memory cells and measured their ability to secrete IL-17 and IFNγ. In line with the EAE results, cblb-deficient Th17 cells showed a significantly increased expression of both cytokines, whereas the IL-17 and IFNγ responses of PKCθ^{-/-} cells were strongly reduced. Of note, the concomitant loss of cblb restored IL-17 partially and IFNγ completely back to wild type levels (Fig. 2C&D). These results indicate that PKCθ and Cbl-b play antagonistic roles in the development of Th17 cell dependent autoimmune responses.

### Example 2.2.: Signaling pathways

Next, the signaling pathways were studied in more detail. As compared to wild type controls, the nuclear translocation and DNA binding of NF-κB and NFAT, key transcription factors regulating IL-2 expression, were strongly augmented in activated cblb^{-/-} CD3+ T cells. Importantly, activation defects in the nuclear translocation and DNA binding of these transcription factors in PKCθ^{-/-} CD3+ T cells were almost completely rescued in the PKCθ/cblb double knockout cells (Fig. 3A&B). These data hint at a T cell-intrinsic antagonistic crosstalk between PKCθ and Cbl-b that converges on the NF-κB and NFAT transactivation pathways. It has been shown that Cbl-b itself becomes ubiquitinated and degraded via the proteasomal pathway after the T cell has been adequately stimulated. Based on the hyporesponsive phenotype of PKCθ-deficient T cells (Fig. 1A-C) and the functional rescues in PKCθ/cblb double-deficient cells, it was investigated whether PKCθ directly regulated Cbl-b activity in primary T cells. Monitoring endogenous Cbl-b protein levels in wild type and PKCθ-deficient CD3+ T cells under different stimulation conditions, it was found that PKCθ-deficient T cells were unable to affect CD28-induced Cbl-b degradation (Fig. 4A&B), a mechanism of post-translational Cbl-b deactivation reported for T cells. Cbl-b mRNA levels were not altered between the genotypes in CD3/CD28 stimulated T cells. Of note, Cbl-b protein levels were not significantly different between wild type and PKCθ-deficient T cells upon CD3 stimulation alone (Fig. 4B), suggesting that proliferation and IL-2 secretion defects of PKCθ^{-/-} T cells might be restricted to CD3/CD28 double stimulation conditions. Indeed, PKCθ-deficient T cells demonstrated the same degree of proliferation and IL-2 secretion as wild type controls when they were activated with anti-CD3 alone, whereas they exhibited a profound activation defect under CD3/CD28 double stimulation conditions (Fig. 4C&D). This clearly indicates that PKCθ is necessary to deliver the CD28 signal for a full T cell response.

### Example 2.3: Interaction between Cbl-b and PKCθ in T cells

Here, it was investigated if these proteins physically interacted; yeast two-hybrid analysis indeed strongly suggested Cbl-b as a PKCθ binding protein. The NH2-terminal region of Cbl-b (aa 1-483, comprising the TKB domain) interacted with PKCθ regulatory domain, the C2-like domain of PKCθ in particular (Table 3).

**Table 3: Specific interaction between PKCθ and Cbl-b in the GAL4 Two-Hybrid System**

| DNA-binding domain hybrid "bait" | Activation-domain hybrid "prey" | Leu-protothrophy |
|---|---|---|
| PKCθ NH₂-terminus | control | - |
| PKCθ COOH-terminus | control | - |
| PKCθ C2-like domain | control | - |
| PKCα NH₂-terminus | control | - |
| PKCθ NH₂-terminus | Cbl-b wt | + |
| PKCθ COOH-terminus | Cbl-b wt | - |
| PKCα NH₂-terminus | Cbl-b wt | - |
| PKCθ C2-like domain | Cbl-b wt | ++ |
| PKCθ NH₂-terminus | Cbl-b NH₂ | + |
| PKCθ COOH-terminus | Cbl-b NH₂ | - |
| PKCα NH₂-terminus | Cbl-b NH₂ | - |
| PKCθ C2-like domain | Cbl-b NH₂ | ++ |
| control | control | - |
| control | control | - |
| control | control | - |
| control | control | - |

Immunoprecipitation analysis confirmed the endogenous interaction between Cbl-b and PKCθ in short-term activated CD3⁺ T cells with a slight increase after CD28 costimulation (appr. 2.4 fold when compared to CD3 single stimulation; Fig. 5A). This inducible interaction appeared to be independent of the catalytic activity of PKCθ, since Cbl-b/PKCθ complex formation remained intact also in panPKC inhibitor-treated cells. Of note, we reproducibly observed a significantly decreased amount of immunoprecipitated PKCθ in cells treated with the panPKC inhibitor. Since the cellular levels of PKCθ were not altered by this inhibitor treatment (see input control in Fig. 5B), a reduced accessibility of the antibody's epitope within inhibitor-bound PKCθ appears to be the most likely explanation. Given the unchanged amounts of co-precipitated Cbl-b despite of strongly decreased levels of inhibitor-bound PKCθ immunoprecipitates (Fig. 5A, compare lanes 2 & 3 with lanes 5 & 6), the relative proportion of Cbl-b attached to enzymatically inhibited PKCθ seemed to be reproducibly higher. A substrate is established to undergo a phosphorylation reaction towards a product that is subsequently released from the kinase. Thus, a plausible explanation of this phenomenon could be the reduced off-rate of Cbl-b once the catalytic activity of PKCθ is suppressed by the inhibitor. This mechanistic explanation is in line with our hypothesis of Cbl-b as a direct substrate of PKCθ in T cells.

Consistently, purified PKCθ was able to phosphorylate this NH2-terminal region of Cbl-b efficiently in vitro (Fig. 7A-C). When identical amounts of recombinant GST fusion subdomains of Cbl-b were used in an in vitro kinase assay, PKCθ readily phosphorylated the Cbl-b subdomain, ranging from aa 215-300. In contrast, PKCθ exhibited no activity in the Cbl-b S282A mutant construct lacking the phosphorylation site at Ser-282 (Fig. 7C). This is consistent with a sequence prediction analysis (performed as described in Fujii et al., Proceedings of the National Academy of Sciences of the United States of America 101 (2004): 13744-13749), identifying (next to S147 and a few additional serine/threonine residues) Ser-282 in the TKB domain of Cbl-b, as putative PKC phosphorylation site. However, since at least two other serine/threonine residues served as phosphorylation sites for PKCθ in vitro, the complex analysis of this multiple serine/threonine phosphorylation status of Cbl-b in intact cells awaits further investigations. By applying a broadly reactive phospho-Ser antibody, a slightly inducible serine phosphorylation of Cbl-b upon PDBu-stimulation in intact T cells (Fig. 7D) could be detected.

### Example 2.4: Cbl-b ubiquitination

Interestingly, and confirming the present model of PKCθ itself being a negative regulator of Cbl-b, the Cbl-b ubiquitination (Ub-Cbl-b) level was significantly enhanced in CA-PKCθ-expressing Jurkat cells (compare Fig. 5C, lane 2 with Fig. 5D, lane 5). When wild type Cbl-b was overexpressed in this experimental system the level of Ub-Cbl-b increased as well (Fig. 5D, lane 6), confirming the specificity of this effect. Importantly, Cbl-b ubiquitination was almost completely abolished in panPKC inhibitor-treated cells (Fig. 5C&D, lanes 3&7), strongly suggesting that the enzymatic activity of the CA-PKCθ mutant was essential for inducing Cbl-b ubiquitination in Jurkat T cells. This result is consistent with the impaired Cbl-b degradation in PKCθ^{-/-} T cells (Fig. 4A&B), independently confirming that PKCθ functions as upstream repressor of Cbl-b by regulating Cbl-b ubiquitination and degradation. Moreover, a selective panPKC inhibitor (Hermann-Kleiter et al., Blood 107 (2006): 4841-4848) on T cells from wild type and cblb-deficient mice was tested, proliferation responses were only completely abrogated in wild type T cells (Fig. 5E), indicating that once the negative signals from Cbl-b were eradicated, T cell activation was no longer fully suppressed by PKC inhibition.
Nevertheless, this only partial resistance of cblb^{-/-} T cells to pharmacological inhibition of PKC reflects established additional PKC functions. Together, this coincides with the scenario that productive CD3/CD28-stimulation requires PKC-mediated suppression of Cbl-b as one rate-limiting element in regulation of the T cell signaling threshold and validates the data obtained with the double-knockout cells.

### Example 3: Discussion

TCR signaling in the absence of co-stimulation results in the induction of anergy. This is one key mechanism leading to immunotolerance, thereby avoiding responses to self-antigens that are usually presented without the costimulatory molecules. In this model, TCR stimulation in the absence of CD28-CD80/CD86 interactions induces the transactivation of the specific target genes of NFAT-regulated immunotolerance, such as E3 ligases itch, grail, and cblb, to maintain the unresponsive state. Indeed, Cbl-b has been shown to regulate the CD28 dependence of T cell activation in vitro and in vivo. cblb^{-/-} T cells are resistant to anergy induction and, consequently, cblb^{-/-} mice show exacerbated autoimmunity. As the functional link between anergy avoidance and CD28 signaling, Cbl-b is ubiquitinated and degraded via the proteasomal pathway once the T cell has been adequately stimulated. However, the molecular mechanism underlying this posttranslational regulation of Cbl-b was not known. In this study, biochemical evidence was provided that PKCθ directly regulates the ubiquitination and degradation of Cbl-b. Numerous studies have established that PKCθ plays an essential role to augment T cell activation by transactivating NF-κB and NFAT. Consequently, IL-2 expression is strongly impaired in PKCθ^{-/-} T cells vitro. One study showed that absence of PKCθ leads to the induction of T cell tolerance and PKCθ-deficient mice are resistant to EAE, as well as to collagen-induced arthritis.

The at least partial genetic rescue of PKCθ^{-/-}-associated defects by the concomitant loss of Cbl-b strongly indicates a critical role for PKCθ and Cbl-b as antagonistic partners in modulating the activation threshold of T cells (Fig. 1A-C, Fig. 2 & Fig. 3). Furthermore, the present novel model explains the opposing phenotypes of the respective mutant T cells: (i) While Cbl-b is an anergy promoting molecule, PKCθ mediates a productive immune response and PKCθ^{-/-} T cells exhibit a tolerance phenotype. (ii) Cbl-b negatively regulates PLCγ1, whereas PKCθ has a positive impact on Ca2⁺ mobilization. (iii) cblb^{-/-} T cells have been shown to display increased adhesion and cell surface binding to ICAM-1, as well as enhanced activity of the small GTPase Rap1, while PKCθ-deficient T cells exhibit impaired Rap1 activation and β2-integrin-mediated T cell adhesion. These data now demonstrate that Cbl-b levels decrease in wild type cells after costimulation, whereas Cbl-b is not downregulated in PKCθ-deficient T cells. Accordingly, Cbl-b ubiquitination is strongly diminished in Jurkat T cells treated with a PKC inhibitor. Due to the reported loss of the lipid-phosphatase PTEN in Jurkat T cells, however, CD28 costimulation is dispensable, thus explaining the observation that Cbl-b ubiquitination appears CD28-independent in Jurkat cells (Fig. 5C&D).

While Cbl-b degradation is dependent on the enzyme activity of PKCθ, the detailed biochemical mechanisms of PKCθ/CD28-dependent Cbl-b ubiquitination remains unknown. Recently, it has been shown that the E3 ubiquitin ligase Nedd4 interacts with Cbl-b and targets it for CD28-dependent ubiquitination and proteasomal degradation. *Nedd4*^{*-*/*-*} T cells thereby display a phenotype remarkably reminiscent of *PKC*θ^{*-*/*-*} T cells. Several studies reported that CD28 signaling is critical for proper translocation of PKCθ into the i-synapse. It is tempting to speculate that PKCθ and Cbl-b colocalize after CD3/CD28 costimulation, being the functional prerequisite for PKCθ-dependent Cbl-b phosphorylation. Thus, previously published data together with our new findings are consistent with the following model: 1) Upon TCR/CD28 costimulation, PKCθ is located in a special subcellular compartment, where it colocalizes with and binds to Cbl-b; 2) PKCθ phosphorylates Cbl-b on Ser-282 (but probably also on other serine/threonine sites), which may induce a conformational change and/or create a new binding site; 3) Nedd4 binds and ubiquitinates Cbl-b, mediating its degradation via the proteasome. It is not quite clear yet why our co-immunoprecipitation data show a PKCθ/Cbl-b interaction already after CD3 stimulation alone. One logical explanation might be that our stimulation protocol does not induce typical i-synapse structures and thus CD3 stimulation is sufficient for some level of PKCθ/Cbl-b colocalization. Nevertheless, the fact remains that Cbl-b is an interacting protein and substrate of PKCθ and PKCθ is required for promoting CD28-induced Cbl-b ubiquitination and degradation. Thusly, loss of PKCθ is sufficient to decrease CD3/CD28 costimulation-dependent T cell responses and deregulation of Cbl-b is one key mechanism behind the hyporesponsiveness of *PKC*θ-deficient T cells (see our schematic model in Fig. 5F).

Altogether, experimental evidence that PKCθ and Cbl-b represent antagonistic participants in one pathway governing the decision-making for tolerance versus productive activation of T cells was provided. Consistently, the activation response defects of PKCθ-deficient T cells in vitro and the resistance of PKCθ^{-/-} mice to antigen-induced autoimmune pathology in vivo can be rescued by the concomitant loss of Cbl-b. This study defines an essential and non-redundant regulatory function of PKCθ in the ubiquitination and subsequent degradation of Cbl-b as a prerequisite for a productive immune response.

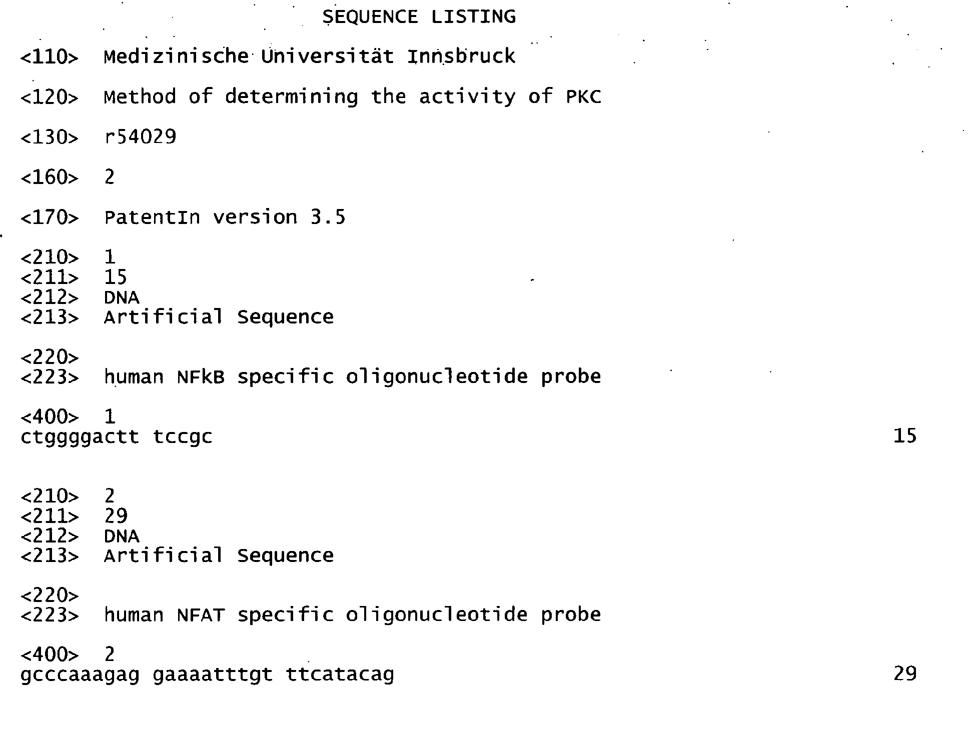

## Claims

1. Method of determining the activity of a protein kinase C (PKC) in a cell or cells of a sample comprising determining the amount of Cbl-b in the cell or cells and correlating the Cbl-b amount to the PKC activity.

2. The method of claim 2, **characterized in that** the step of correlating the Cbl-b amount to the PKC activity comprises determining standard values of different PKC activities and the resulting Cbl-b amount in a cell or cells and comparing the standard values with the Cbl-b amount of the sample.

3. The method of claim 1 or 2, **characterized in that** the cells are eukaryotic cells, preferably of a mammal or bird, in particular preferred of a rodent or primate, even more preferred of a human, mouse, rat, hamster, chimpanzee or pig.

4. The method of any one of claims 1 to 3, **characterized in that** determining the Cbl-b amount comprises determining the amount of Cbl-b mRNA.

5. The method of any one of claims 1 to 4, **characterized in that** determining the Cbl-b amount comprises determining the amount of Cbl-b protein, preferably further ubiquitinated Cbl-b protein, phosphorylated Cbl-b protein.

6. The method of any one of claims 1 to 5, **characterized in that** PKC comprises the PKC-theta isoform.

7. The method of any one of claims 1 to 6, **characterized in that** the cells or cell of the sample are contacted with a drug candidate before or during the Cbl-b amount is determined.

8. The method of claim 7, **characterized in that** the Cbl-b amount after contact with the drug candidate is correlated to an increase or decrease of PKC activity due to the drug candidate in reference to a cell or cells without contact to the drug candidate.

9. The method of any one of claims 1 to 8, **characterized in that** the intracellular Cbl-b amount is determined, preferably in single cells, in particular preferred by flow-cytometry.

10. The method of claim 9, **characterized in that** the intracellular Cbl-b amount is determined by introducing anti-Cbl-b antibodies into the cell or cells, preferably an antibody which is suitable to detect intracellular Cbl-b after cell fixation.

11. The method of claim 10, **characterized in that** the antibody recognizes an epitope within the C-terminal 300 amino acids of Cbl-b, preferably within the C-terminal 250 amino acids of Cbl-b.

12. The method of prognosing, evaluating or monitoring the efficacy of a PKC-theta modulation therapy, preferably a PKC-theta inhibitor therapy, in a patient comprising determining the amount of Cbl-b in a cell or cells in a sample of the patient to whom the PKC-theta modulator is administered, preferably wherein Cbl-b is determined according to a method of any one of claims 1 to 11.

13. The method of claim 12, **characterized in that** the patient suffers from a inflammatory and/or immunological diseases, preferably Th17-mediated inflammatory or immunological diseases, such as autoimmune disorders, or a Th1 and/or Th2-mediated allergic inflammation, in particular autoimmune encephalitis, multiple sclerosis, arthritis, transplant rejection, inflammatory bowel disease, psoriasis.

14. Use of the method according to any one of claims 1 to 11 to identify drugs for the treatment of inflammatory and/or immunological diseases, preferably Th17-mediated inflammatory or immunological diseases, such as autoimmune disorders, or a Th1 and/or Th2-mediated allergic inflammation, in particular autoimmune encephalitis, multiple sclerosis, arthritis, transplant rejection, inflammatory bowel disease, psoriasis.

15. Use according to claim 14, further comprising preparing a pharmaceutical composition with the drug for the treatment of an inflammatory and/or immunological disease.
